# EUROPÄISCHE PATENTSCHRIFT

(11) **EP 0 644 874 B1**
(45) Veröffentlichungstag und Bekanntmachung des Hinweises auf die Patenterteilung: **21.02.1996**
(21) Anmeldenummer: 93912868.2
(22) Anmeldetag: 03.06.1993
(51) Int. Cl.: C07C 251/54, C07C 259/06

(54) **COPOLYMERISIERBARE OXIMETHER UND DIESE ENTHALTENDE COPOLYMERISATE**
COPOLYMERIZABLE OXIME ETHERS AND COPOLYMERS CONTAINING THEM
ETHERS D'OXIMES COPOLYMERES ET COPOLYMERISATS LES RENFERMANT

(30) Priorität: 13.06.1992 DE 4219385
(43) Veröffentlichungstag der Anmeldung: 29.03.1995
(73) Patentinhaber: BASF Aktiengesellschaft, D-67063 Ludwigshafen (DE)
(72) Erfinder: BOTT, Kaspar, D-6800 Mannhein 1 (DE); GOETZ, Norbert, D-6520 Worms 1 (DE); BAUER, Gerhard, D-6940 Weinheim (DE); AYDIN, Oral, D-6800 Mannheim 1 (DE)
(86) Internationale Anmeldenummer: EP9301396
(87) Internationale Veröffentlichungsnummer: WO9325519

(56) Entgegenhaltungen:
- EP-A- 3 516
- EP-A- 0 035 291
- EP-A- 0 206 059
- DE-A- 3 807 555
- US-A- 4 396 738

## Beschreibung

Die Erfindung betrifft Oximether der allgemeinen Formel 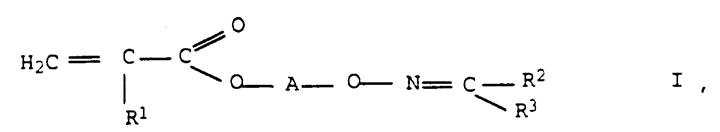 worin A für ein zweiwertiges Bindeglied steht, R¹ ein Wasserstoffatom oder eine C₁- bis C₄-Alkylgruppe sein kann und R und R³ unabhängig voneinander für einen C₁- bis C₁₀-Alkyl-, einen C₁-C₁₀-Alkoxy-, einen C₅- bis C₁₀-Cycloalkyl- oder einen C₅- bis C₁₀-Arylrest, welche auch 1 - 3 nicht benachbarte Stickstoff-, Sauerstoff- oder Schwefelatome als Heteroatome in der Kohlenstoffkette oder im Kohlenstoffring enthalten können und durch ein bis drei C₁- bis C₄-Alkyl- oder -Alkoxygruppen substituiert sein können, stehen, R oder R³ für ein Wasserstoffatom stehen können oder R und R³ gemeinsam eine Brücke aus 3 bis 14 Kohlenstoffatomen bilden, wobei ein Teil der Kohlenstoffatome auch Bestandteil eines aromatischen Rings sein kann.

Bei Copolymerisaten, welche in Beschichtungsmitteln oder Klebstoffen Verwendung finden, handelt es sich vielfach um vernetzungsfahige Copolymerisate. Durch eine Vernetzung können z.B. Schutzüberzüge oder Klebstoffbeschichtungen mit guten elastischen Eigenschaften, hoher Kohäsion, hoher Chemikalien- und Lösemittelbeständigkeit erhalten werden.

Zur Vernetzung wird den Copolymerisaten im allgemeinen ein Vernetzungsmittel zugesetzt, das mit funktionellen Gruppen im Copolymerisat reagiert. Mögliche Vernetzungsmittel sind z.B. Polyisocyanate, welche mit Hydroxylgruppen oder Aminogruppen reagieren.

Aus der DE-A-35 21 618 sind entsprechende wäßrige Klebstoffzubereitungen bekannt, bei denen in Wasser dispergierte Polyisocyanate wäßrigen Dispersionen radikalisch polymerisierter Copolymerisate als Vernetzungsmittel zugesetzt werden. Ähnliche Klebstoffzubereitungen sind auch in der US-A-43 96 738 und DE-A-31 12 117 beschrieben.

Nachteilig bei diesen wäßrigen Zubereitungen ist jedoch die mangelnde Lagerstabilität. Das Polyisocyanat darf daher erst kurz vor seiner Verwendung als Vernetzungshilfsmittel in Wasser dispergiert und mit dem Copolymerisat gemischt werden.

Eine erhöhte Lagerstabilität kann durch Umsetzung der Isocyanatgruppen mit Blockierungsmitteln, z.B. Oximen, Caprolactam, Phenolen, Maleinsäuredialkylestern erreicht werden. Die erhaltenen sog. blockierten Polyisocyante hydrolysieren in wäßriger Dispersion nur noch in untergeordnetem Ausmaß.

Gegenstand der DE-A-38 07 555 ist ein solches, mit Oximen blockiertes Diisocyanat, welches in Wasser dispergiert wird und sich als Zusatz für in Wasser dispergierte Polymerisate eignet.

Vernetzungsreaktionen treten jedoch erst nach Abspaltung des Blockierungsmittels bei Temperaturen ab ca. 130°C auf.

Bisher bekannte wäßrige Klebstoffzubereitungen mit Polyisocyanaten als Vernetzungsmittel sind daher entweder nicht lagerstabil und können daher nur als 2-Komponentensystem Verwendung finden oder vernetzen erst bei hohen Temperaturen.

Lagerstabile, bei Raumtemperatur nach Entfernen des Lösungsmittels vernetzende wäßrige Dispersionen sind aus der EP-A-3516 bekannt. Diese Dispersionen enthalten Polyhydrazide, welche mit im Copolymerisat einpolymerisieren Monomeren mit Carbonylgruppen reagieren.

Es ist grundsätzlich erwünscht, weitere lagerstabile, bei Raumtemperatur vernetzende Dispersionen zu entwickeln, um Alternativen zur Polyhydrazidvernetzung zur Verfügung zu stellen.

Aufgabe der vorliegenden Erfindung waren daher vernetzbare Copolymerisate, welche in Dispersion oder Lösung auch in Gegenwart eines Vernetzungsmittels lagerstabil sind und bei Raumtemperatur vernetzt werden können.

Demgemäß wurden die oben definierten copolymerisierbaren Oximether und ein Verfahren zu ihrer Herstellung gefunden.

Des weiteren wurden Copolymerisate, welche die copolymerisierbaren Oximether enthalten und die Verwendung der Copolymerisate als Beschichtungsmittel oder Klebstoff gefunden.

Die Copolymerisate, welche die copolymerisierbaren Oximether enthalten, vernetzen mit Aldehyd- oder Ketogruppen enthaltenden Vernetzungsmitteln wahrscheinlich nach folgendem Mechanismus: 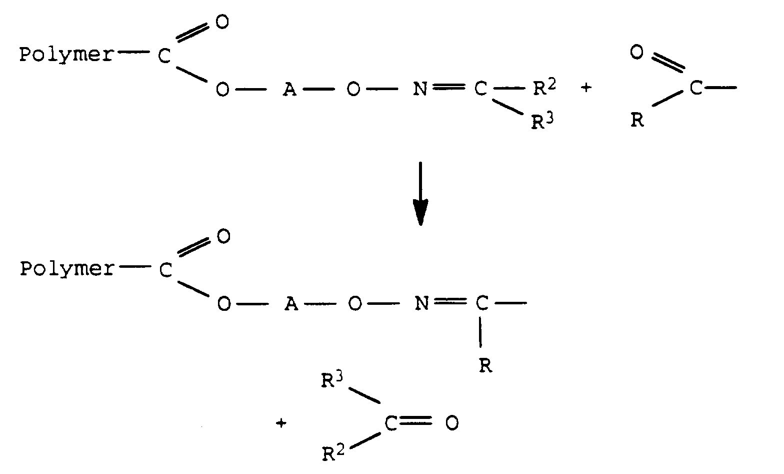 Bei der Variablen A in der allgemeinen Formel I handelt es sich bevorzugt um eine lineare oder verzweigte Kohlenwasserstoffkette aus 2 bis 12, insbesondere 2 bis 8 Kohlenstoffatomen, welche gegebenenfalls durch bis 3, insbesondere 1 - 2 nicht benachbarte Sauerstoffatome oder einen C₅-C₁₀-Cycloalkylen- oder einen C₅-C₁₀-Arylenring unterbrochen sein kann. Besonders bevorzugt handelt es sich um eine lineare oder verzweigte Kohlenwasserstoffkette aus 2 bis 8 Kohlenstoffatomen.

R¹ steht bevorzugt für ein Wasserstoffatom oder eine Methylgruppe; R und R³ stehen bevorzugt unabhängig voneinander für ein Wasserstoffatom, eine C₁- bis C₆-Alkylgruppe, C₁- bis C₆-Alkoxygruppe oder ein C₅-C₁₀-Arylrest, insbesondere einen Phenylring. Im Falle des Wasserstoffatoms kann nur einer der beiden Reste R und R³ ein Wasserstoffatom sein.

Die copolymerisierbaren Oximether können z.B. durch Umesterung von Oximetheralkoholen der Formel 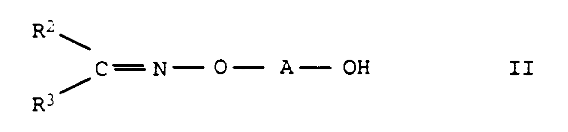 mit Alkyl(meth-)acrylaten, insbesondere den Methyl- oder Ethyl(meth-)acrylaten hergestellt werden.

Vorteilhaft ist jedoch die Umsetzung von Oximetheralkoholen mit (meth) acrylsäurechloriden oder (Meth)acrylsäureanhydriden der Formeln 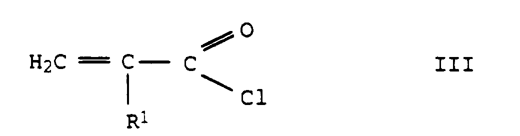 oder 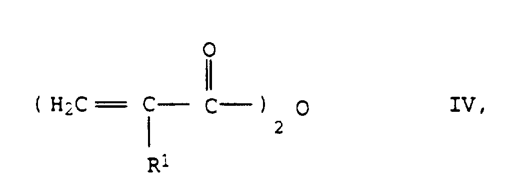 da die Reaktionszeiten kurz und die Reaktionstemperaturen niedrig gehalten werden können und so unerwünschte Nebenreaktionen wie Polymerisation und Zersetzung weitgehend vermieden werden.

Die Umsetzung der Oximetheralkohole II mit (Meth-)acrylsäurechloriden III erfolgt bevorzugt unter Kühlung der Reaktionsmischung bei Temperaturen zwischen 0 und 50°C, insbesondere 10 bis 30°C. Im Falle der (Meth)acrylsäureanhydride IV sollte die Temperatur oberhalb von 60°C liegen. Die Umsetzung kann in einem organischen Lösungsmittel oder auch lösungsmittelfrei erfolgen. Als Lösungsmittel in Betracht zu ziehen sind z.B. Cyclohexan, Methylenchlorid, Diethylether, Methyl-tert-butylether, Ethylenchlorid.

Insbesondere sind auch in Hinsicht auf die weitere Aufarbeitung Ether, z.B. Diethylether als Lösungsmittel geeignet. Die Umsetzung wird bevorzugt basisch katalysiert. Als Basen bzw. basisch reagierende Verbindungen können z.B. tertiäre Stickstoffverbindungen wie Triethylamin eingesetzt werden. Die Base oder basisch reagierende Verbindung wird vorzugsweise in äquimolaren Mengen bezogen auf den Oximetheralkohol eingesetzt.

Nach Beendigung der Umsetzung kann die Reaktionsmischung z.B. mit einer wäßrigen Natriumcarbonatlösung gewaschen und der erhaltene copolymerisierbare Oximether gegebenenfalls nach Abtrennung des Lösungsmittels durch Destillation gereinigt werden.

Die eingesetzten Oximetheralkohole sind nach bekannten Verfahren erhältlich durch Umsetzung von Oximen mit Ethylenoxid, Propylenoxid oder mit Halogenalkoholen in Gegenwart einer Base.

Die copolymerisierbaren Oximether (im folgenden auch als Monomere a) bezeichnet) können nach üblichen Verfahren der radikalischen Polymerisation mit ethylenisch ungesättigten Monomeren copolymerisiert werden.

Für eine ausreichende Vernetzungsfähigkeit der erhaltenen Copolymerisate sollte der Gehalt an einpolymerisierten Oximethern a) mindestens 0,01 Gew.-% betragen. Ein Gehalt über 30 Gew.-% ist im allgemeinen nicht notwendig.

Vorzugsweise ist der Gehalt an einpolymerisierten Oximethern im Copolymerisat 0,1 bis 10, besonders bevorzugt 0,1 bis 5 Gew.-%.

Die Copolymerisate enthalten als Hauptmonomere b) 30 - 99,99, vorzugsweise 70 bis 99,9, besonders bevorzugt 85 bis 99,9 Gew.-% eines Monomeren ausgewählt aus C₁-C₂₀-Alkyl(meth)acrylaten, Vinylestern von bis zu 20 C-Atome enthaltenden Carbonsäuren, Vinylaromaten mit bis zu 20 C-Atomen, ethylenisch ungesättigte Nitrile, Vinylhalogenide und nichtaromatischen Kohlenwasserstoffen mit mindestens 2 konjugierten Doppelbindungen.

Als Hauptmonomere zu nennen sind z.B. (Meth)-acrylsäurealkylester mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäure-alkylester geeignet.

Vinylester von Carbonsäuren mit 1 bis 20 C-Atomen sind z.B. Vinyllaurat, -stearat, Vinylpropionat und Vinylacetat.

Als vinylaromatische Verbindungen kommen Vinyltoluol, α- und p-Styrol, α-Butylstyrol, 4-n-Butylstyrol, 4-n-Decylstyrol und vorzugsweise Styrol in Betracht. Beispiele für Nitrile sind Acrylnitril und Methacrylnitril.

Die Vinylhalogenide sind mit Chlor, Fluor oder Brom substituierte ethylenisch ungesättigte Verbindungen, bevorzugt Vinylchlorid und Vinylidenchlorid.

Als nicht aromatische Kohlenwasserstoffe mit 2 bis 8 C-Atomen und mindestens zwei olefinischen Doppelbindungen seinen Butadien, Isopren, und Chloropren genannt.

Die Hauptmonomeren werden auch vorzugsweise im Gemisch eingesetzt.

Die Copolymerisate können weiterhin auch Monomere mit mindestens einer Aldehyd- oder Ketogruppe (Monomere c)) enthalten.

Vorzugsweise handelt es sich um Monomere mit ein oder zwei Aldehyd-, bzw. Ketogruppen oder einer Aldehyd- und einer Ketogruppe und einer radikalisch polymerisierbaren olefinischen Doppelbindung.

In Betracht kommen z.B. Acrolein, Methacrolein, Vinylalkylketone mit 1 bis 20, vorzugsweise 1 bis 10 Kohlenstoffatomen im Alkylrest, Formylstyrol, (Meth-)acrylsäurealkylester mit ein oder zwei Keto- oder Aldehyd-, bzw. einer Aldehyd- und einer Ketogruppe im Alkylrest, wobei der Alkylrest vorzugsweise insgesamt 3 bis 10 Kohlenstoffatome umfaßt, z.B. (Meth)-acryloxyalkylpropanale, wie sie in der DE-A-27 22 097 beschrieben sind. Des weiteren eignen sich auch N-Oxoalkyl(meth)acrylamide wie sie z.B. aus der US-A-4 226 007, der DE-A-20 61 213 oder DE-A-22 07 209 bekannt sind.

Besonders bevorzugt sind Acetoacetyl(meth)acrylat, Acetoacetoxyethyl(meth)acrylat und insbesondere Diacetonacrylamid.

Der Gehalt dieser Monomeren beträgt im allgemeinen zwischen 0 und 30 Gew.-%, insbesondere zwischen 0 und 10, besonders bevorzugt zwischen 0 und 5 Gew.-%.

Das Copolymerisat kann selbst- oder fremdvernetzbar sein. Im Falle der Selbstvernetzbarkeit enthält es sowohl copolymerisierbare Oximether als auch Monomere mit mindestens einer Keto- oder Aldehydgruppe. Die Vernetzung des Copolymerisats tritt dann ohne Zusatz eines Vernetzungsmittels durch Reaktion der Oximgruppe mit den Keto- oder Aldehydgruppe im gleichen Copolymerisat ein.

Der Gehalt am Monomer mit mindestens einer Keto- oder Aldehydgruppe c) im Copolymerisat sollte dann vorzugsweise mindestens 0,1 Gew.-% betragen. Die maximal mögliche Menge des Hauptmonomeren reduziert sich dann um 0,1 Gew.-%.

Als weitere von den Monomeren a) bis c) verschiedene Monomere d), welche im Copolymerisat enthalten sein können, sind z.B. Ester der Acryl- und Methacrylsäure von Alkoholen mit 1 bis 20 C-Atomen, die außer dem Sauerstoffatom in der Alkoholgruppe mindestens ein weiteres Heteroatom enthalten und/oder die einen aliphatischen oder aromatischen Ring enthalten, wie 2-Ethoxyethylacrylat, 2-Butoxyethyl(meth)acrylat, Dimethylaminoethyl(meth)acrylat, Diethylaminoethyl(meth)acrylat, (Meth)acrylsäurearyl-, -alkaryl- oder Cycloalkylester, wie Cyclohexyl(meth)acrylat, Phenylethyl(meth)-acrylat, Phenylpropyl(meth)acrylat- oder Acrylsäureester von heterocyclischen Alkoholen wie Furfuryl(meth)acrylat genannt.

Darüber hinaus kommen noch weitere Monomere wie (Meth)acrylamid, sowie deren am Stickstoff mit C₁-C₄-Alkyl substituierten Derivate, in Betracht.

Von Bedeutung sind auch hydroxyfunktionelle Monomere, z.B. (Meth)acrylsäure-C₁-C₁₅-alkylester, welche durch ein oder zwei Hydroxygruppen substituiert sind. Insbesondere von Bedeutung als hydroxyfunktionelle Comonomere sind (Meth)acrylsäure-C₁-C₈-Hydroxyalkylester, wie n-Hydroxyethyl-, n-Hydroxypropyl- oder n-Hydroxybutyl(meth)acrylat.

Die Mitverwendung von Comonomeren mit salzbildenden Gruppen empfiehlt sich für die Herstellung selbstdispergierbarer Copolymerisate, die z.B. für wäßrige Sekundärdispersionen geeignet sind. Monomere mit salzbildenden Gruppen sind insbesondere Itaconsäure, Acrylsäure und Methacrylsäure.

Der Gewichtsanteil der weiteren Comonomeren im Copolymerisat kann 0 bis 50 %, vorzugsweise 0 bis 20 % und ganz besonders bevorzugt 0 bis 10 Gew.-% betragen.

Die Herstellung des Copolymerisats A) erfolgt durch radikalische Polymerisation. Geeignete Polymerisationsmethoden, wie Substanz-, Lösungs-, Suspensions- oder Emulsionspolymerisation sind dem Fachmann bekannt.

Vorzugsweise wird das Copolymerisat durch Lösungspolymerisation mit anschließender Dispergierung in Wasser oder besonders bevorzugt durch Emulsionspolymerisation hergestellt.

Die Comonomeren können bei der Emulsionspolymerisation wie üblich in Gegenwart eines wasserlöslichen Initiators und eines Emulgators bei vorzugsweise 30 bis 95°C polymerisiert werden.

Geeignete Initiatoren sind z.B. Natrium-, Kalium- und Ammoniumpersulfat, tert.-Butylhydroperoxide, wasserlösliche Azoverbindungen oder auch Redoxinitiatoren.

Als Emulgatoren dienen z.B. Alkalisalze von längerkettigen Fettsäuren, Alkylsulfate, Alkylsulfonate, alkylierte Arylsulfonate oder alkylierte Biphenylethersulfonate. Des weiteren kommen als Emulgatoren Umsetzungsprodukte von Alkylenoxiden, insbesondere Ethylen- oder Propylenoxid mit Fettalkoholen, -säuren oder Phenol, bzw. Alkylphenolen in Betracht.

Im Falle von wäßrigen Sekundärdispersionen wird das Copolymerisat zunächst durch Lösungspolymerisation in einem organischen Lösungsmittel hergestellt und anschließend unter Zugabe von Salzbildnern, z.B. von Ammoniak zu Carbonsäuregruppen enthaltenden Copolymerisaten, in Wasser ohne Verwendung eines Emulgators oder Dispergierhilfsmittels dispergiert. Das organische Lösungsmittel kann abdestilliert werden. Die Herstellung von wäßrigen Sekundär-dispersionen ist dem Fachmann bekannt und z.B. in der DE-A-37 20 860 beschrieben.

Zur Einstellung des Molekulargewichts können bei der Polymerisation Regler eingesetzt werden. Geeignet sind z.B. -SH enthaltende Verbindungen wie Mercaptoethanol, Mercaptopropanol, Thiophenol, Thioglycerin, Thioglykolsäureethylester, Thioglykolsäuremethylester und tert.-Dodecylmercaptan.

Die Art und Menge der Comonomeren wird zweckdienlicherweise so gewählt, daß das erhaltene Copolymerisat eine Glasübergangstemperatur zwischen bevorzugt -60 und +140°C, besonders bevorzugt -30 und +80°C und ganz besonders bevorzugt, vor allem im Falle der Verwendung als Klebstoff, zwischen -30 und +20°C hat. Die Glasübergangstemperatur des Copolymerisats läßt sich nach üblichen Methoden wie Differentialthermoanalyse oder Differential Scanning Calonimetrie (s. z.B. ASTM 3418/82, sog. "midpoint temperature") bestimmen.

Im Falle, daß das Copolymerisat nicht selbstvernetzend ist, also keine Monomere c) enthält, kann dem Copolymerisat geeigneterweise für die Vernetzung ein Vernetzungsmittel zugegeben werden. Bei dem Vernetzungsmittel handelt es sich üblicherweise um eine Verbindung, welche mindestens zwei Keto- oder Aldehydgruppen, bzw. mindestens eine Keto- und eine Aldehydgruppe enthält.

Geeignet sind z.B. als Vernetzungsmittel insbesondere auch radikalische Copolymerisate - im folgenden auch polymeres Vernetzungsmittel genannt - welche voranstehend genannte Monomere c) einpolymerisiert enthalten.

In Betracht kommen z.B. polymere Vernetzungsmittel, die aus 30 - 99,9 Gew.-%, vorzugsweise 70 - 99,9 Gew.-% der Monomeren b), 0,1 - 30 Gew.-%, vorzugsweise 0,1 - 10 Gew.-% der Monomeren c) und 0 - 50 Gew.-%, vorzugsweise 0 - 20 Gew.-% der Monomeren d) aufgebaut sind. Über die Art der Monomeren, die Glasübergangstemperatur und die Herstellung gilt vorzugsweise das voranstehend über obige Copolymerisate gesagte.

Das Vernetzungsmittel, soweit gewünscht, wird vorzugsweise zur Lösung oder Dispersion der Copolymerisate gegeben.

Es ist jedoch auch möglich, das Copolymerisat und das Vernetzungsmittel erst bei der Anwendung, z.B. der Beschichtung von Oberflächen zusammenzubringen. Dazu könnte auf die Oberfläche z.B. zunächst das Vernetzungsmittel als sog. Primer aufgebracht werden und anschließend die Beschichtung mit der Dispersion oder Lösung der Copolymerisate erfolgt.

Die Lösung oder Dispersion der erfindungsgemäßen Copolymerisate eigent sich z.B. für die Verwendung als Anstrich- und Beschichtungsmittel für unterschiedliche Substrate mit Kunststoff-, Holz- oder Metalloberflächen oder z.B. für Textilien, Vliesstoffe, Leder oder Papier. Sie eignen sich ebenfalls für Anwendungen in der Bauchemie z.B. als Klebstoffe, Dichtungsmassen, Bindemittel oder ähnliches.

Die Dispersionen als Lösungen können je nach Verwendungszweck noch übliche Hilfs- und Zusatzmittel enthalten. Hierzu gehören beispielsweise Füllstoffe wie Quarzmehl, Quarzsand, hochdisperse Kieselsäure, Schwerspat, Calciumcarbonat, Kreide, Dolomit oder Talkum, die oft zusammen mit geeigneten Netzmitteln wie z.B. Polyphosphaten wie Natriumhexamethaphosphat, Naphthalinsulfonsäure, Ammonium- oder Natriumpolyacrylsäuresalze eingesetzt werden, wobei die Netzmittel im allgemeinen von 0,2 bis 0,6 Gew.-%, bezogen auf den Füllstoff, zugesetzt werden.

Auch Fungizide zur Konservierung können zugesetzt werden. Diese kommen im allgemeinen in Mengen von 0,02 bis 1 Gew.-%, bezogen auf die Dispersionen oder Lösungen zum Einsatz. Geeignete Fungizide sind beispielsweise Phenol- oder Kresol-Derivate oder zinnorganische Verbindungen.

Besonders eignen sich die Dispersionen oder Lösungen auch als Dichtstoff- oder Klebstoffzubereitungen, insbesondere als Kaschierklebstoff zur Herstellung von Verbundfolien und Glanzfolien. Als solche können sie neben obengenannten Zusatzstoffen noch spezielle, in der Klebstofftechnologie übliche Hilfs- und Zusatzmittel enthalten. Hierzu gehören beispielsweise Verdickungsmittel, Weichmacher oder auch klebrigmachende Harze wie z.B. Naturharze oder modifizierte Harze wie Kolophoniumester oder synthetische Harze wie Phthalatharze.

Die Dispersionen oder Lösungen der selbstvernetzenden oder fremdvernetzenden Copolymerisate, welche noch ein Vernetzungsmittel enthalten, sind lagerstabil. Die Vernetzung tritt bereits bei Raumtemperatur mit Verflüchtigung des Lösungsmittels ein.

Die mit diesen Dispersionen oder Lösungen hergestellten Beschichtungen oder Verklebungen weisen eine gute Chemikalien- oder Lösungsmittelbeständigkeit und eine gute innere Festigkeit (Kohäsion) auf.

### Beispiele

### Herstellung der copolymerisierbaren Oximether

### Beispiel 1

- Apparatur:: 1-1-Rührkolben aus Glas mit Rückflußkühler, Tropftrichter und CaCl₂-Rohr.

Zu einer Mischung von 400 ml Diethylether, 58,5 g (0,50 mol) O-(2-Hydroxyethyl)-aceton-oxim und 53,0 g (0,52 mol) Triethylamin wurde unter Rühren eine Lösung von 47,1 g (0,52 mol) Acrylsäurechlorid in 100 ml Diethylether zugetropft. Die Temperatur der Reaktionsmischung wurde dabei durch Kühlung mit Eiswasser auf 20 bis 22°C gehalten. Anschließend wurde noch 4 h bei 25°C gerührt.

Zum Kolbeninhalt wurde dann 1 l 5-proz. Sodalösung gegeben und 20 min gerührt. Danach wurde die (obere) Ether-Phase abgetrennt, bei 20 mbar und 25°C eingedampft und der Eindampfrückstand ohne Destillationskolonne im Vakuum destilliert (siehe Tab. 1).

Dabei fielen 74 g (87 % Ausbeute) reiner Acetonoxim(2-acroyloxy-ethyl)ether an, der anhand seines H-NMR-Spektrums in D₆-DMSO identifiziert wurde: δ = 1,75 (s, 3H, CH3); 1,79 (s, 3H, CH3); 4,15 (t, 2H, -CH₂-); 4,30 (t, 2H, -CH2-); 5,95 (d, 1H, (β)=CH-); 6,20 (dd, 1H, (α)=CH-) und 6,35 (d, 1H, (β) =CH-).

### Beispiel 2

Zu einer Mischung aus 152 g (1,30 mol) O-(2-Hydroxyethyl)-acetonoxim, 131,3 g (1,30 mol) Triethylamin und 0,40 g Phenothiazin (Stabilisator) wurden bei 60°C 209,4 g (1,36 mol) Methacrylsäureanhydrid zugetropft (45 min Eintopfzeit, 45 min Nachreaktionszeit). Der Kolbeninhalt wurde auf Raumtemperatur abgekühlt und mit einer Lösung von 179,4 g (1,30 mol) Kaliumcarbonat in 1 l Wasser geschüttelt. Die obere Phase wurde in einem Scheidetrichter abgetrennt und im Vakuum ohne Destillationskolonne destilliert. Dabei wurden 209 g (87 % Ausbeute) reiner Acetonoxim-(2-methacroyloxyethyl)-ether erhalten.
- Elementaranalyse:: berechnet C 58,36 H 8,16 O 25,91
gefunden C 58,0 H 8,2 O 25,8

### Beispiele 3 bis 11

Die Herstellung der weiteren Oximether erfolgte analog zu Beispiel 1 bzw. 2 (s. Tabelle 1).

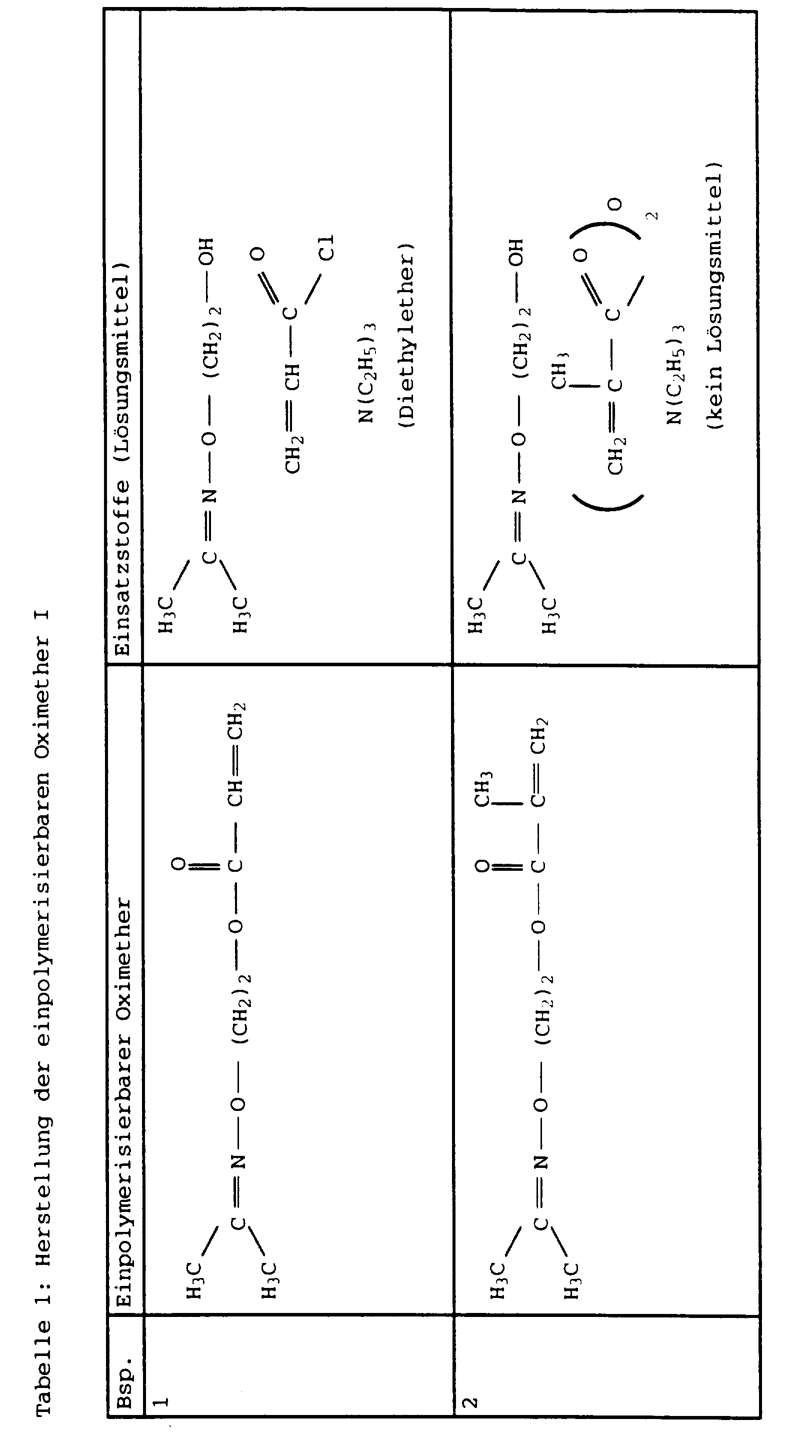

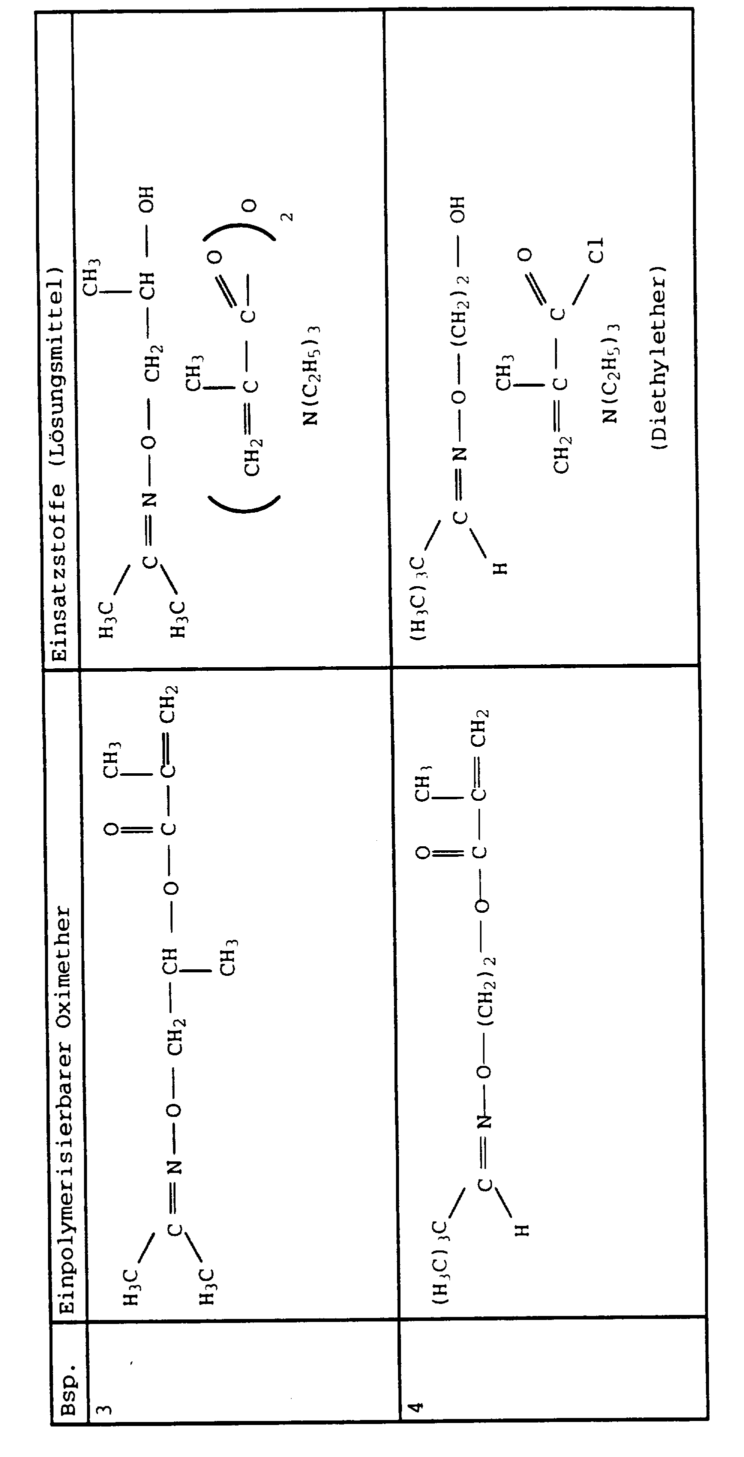

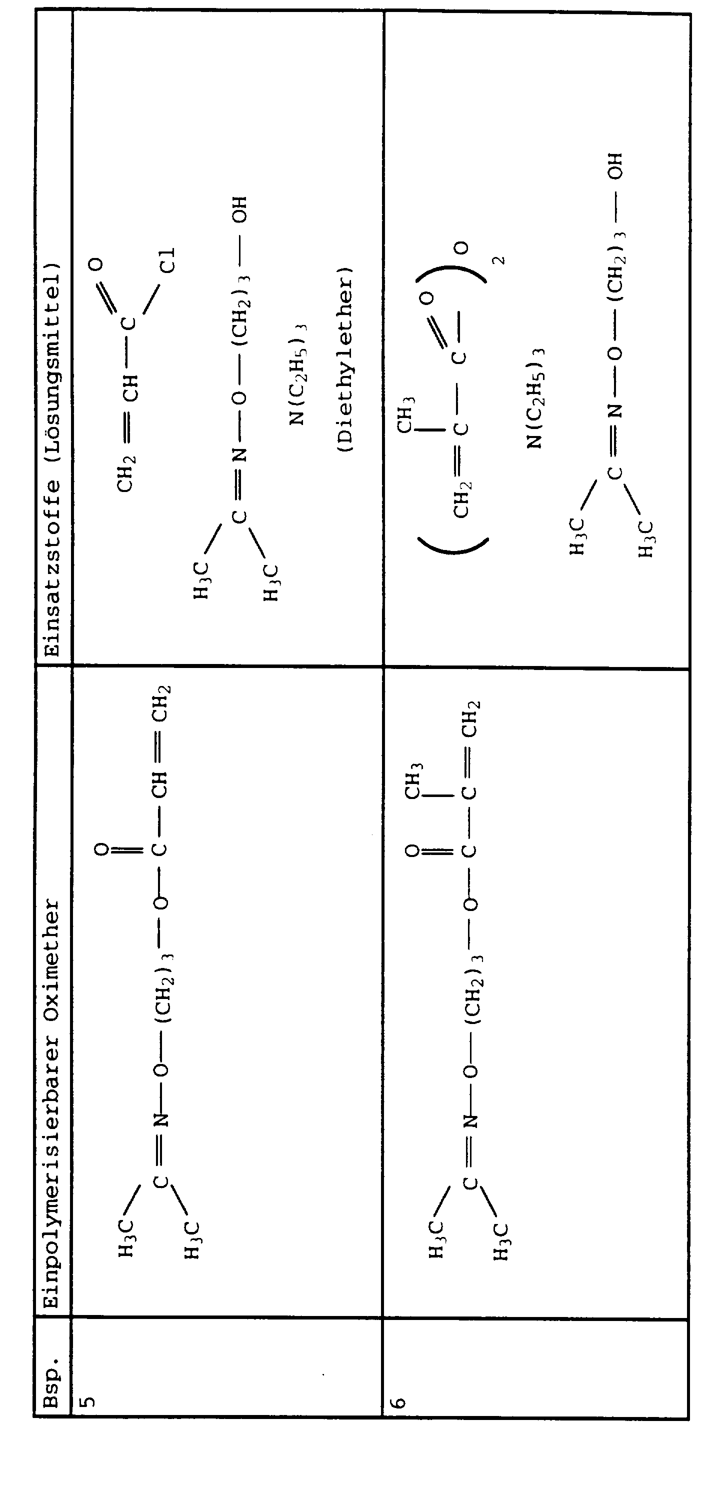

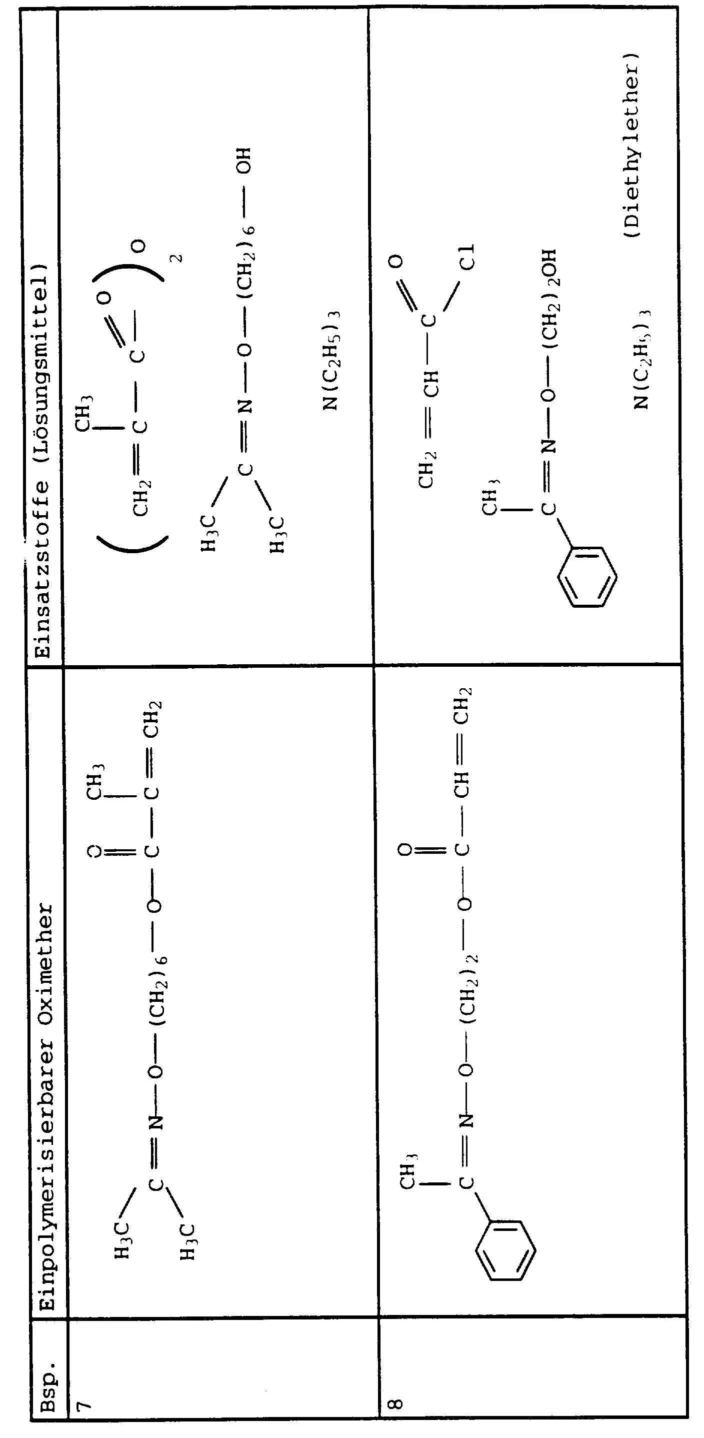

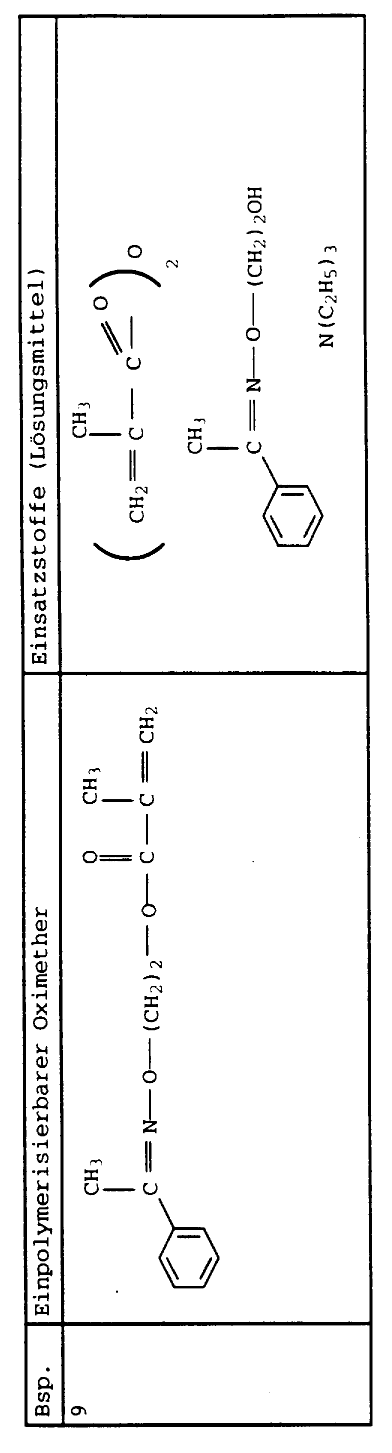

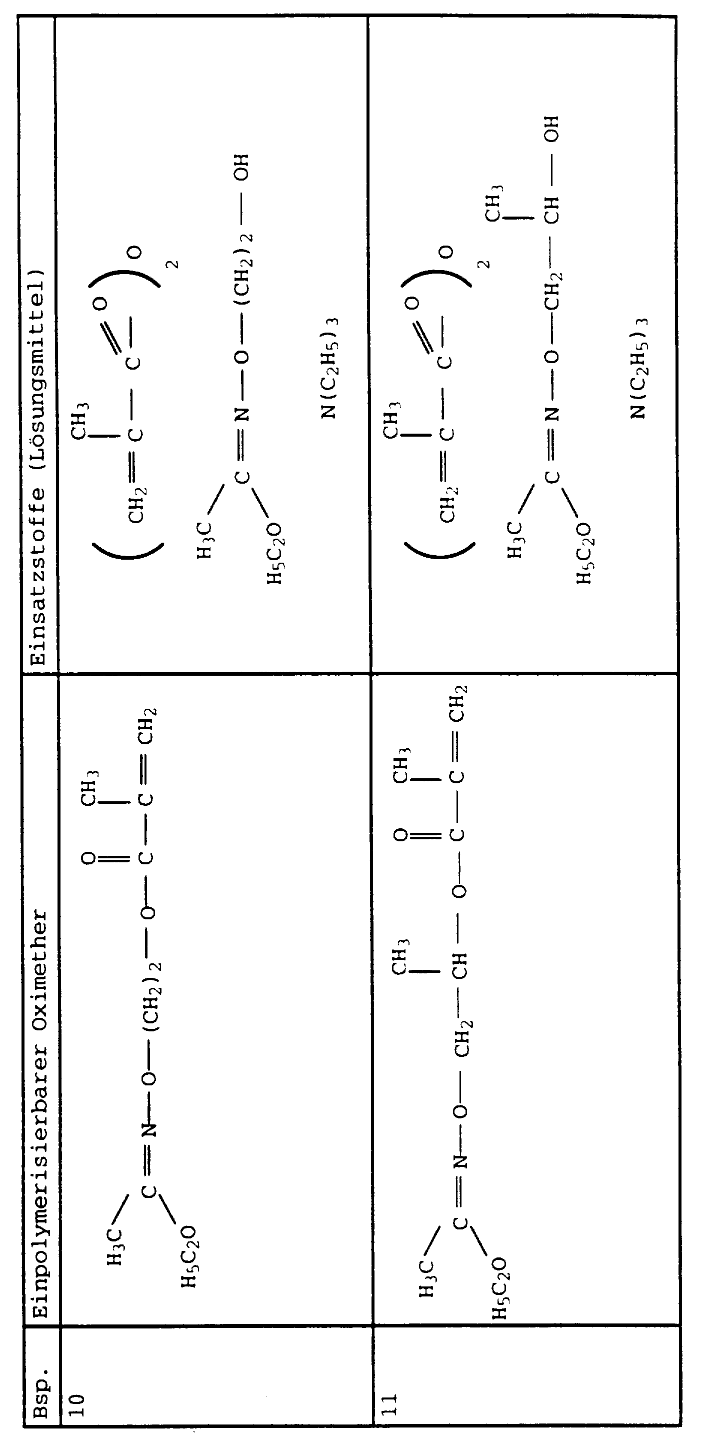

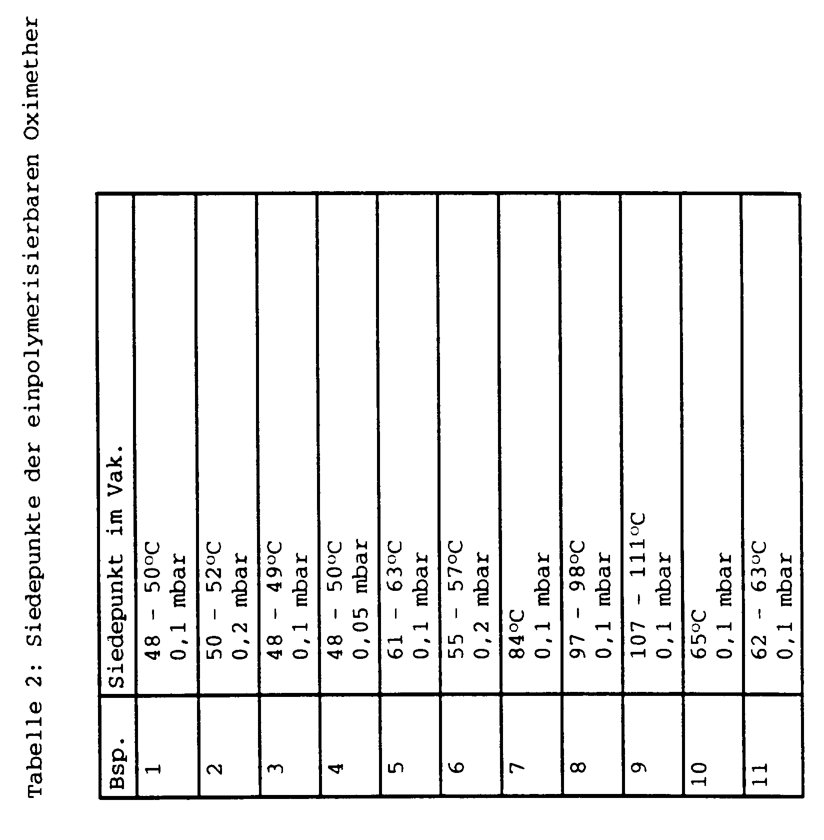

Herstellung von polymeren Vernetzungsmitteln, welche Carbonylgruppen enthalten

### Vernetzungsmittel 1 (V1)

In einem Reaktionsgefäß mit Rührer und zwei Zulaufgefäßen (Zulauf 1 und Zulauf 2) wurden 200 g entsalztes Wasser, 37 g des Zulaufs 1 (siehe unten) und 20 g des Zulaufs 2 vorgelegt und auf 85°C erwärmt. Nach 15 Minuten wurde innerhalb von 2 h gleichmäßig der Zulauf 1 sowie innerhalb von 2,5 h gleichmäßig der Zulauf 2 zum Reaktionsgefäß zugegeben. Nach der letzten Initiator-Zugabe (Zulauf 2) wurde die Dispersion noch 1 Stunde bei 85°C gerührt.

### Zulauf 1: (dieser Zulauf wird während der Polymerisation gerührt)

- 107,5 g: entsalztes Wasser
- 400 g: Ethylacrylat
- 90 g: Methylmethacrylat
- 50 g: 20 gew.-%ige wäßrige Diacetonacrylamid-Lösung
- 50 g: 20 gew.-%ige Lösung des Natriumsalzes vom p-Dodecyldiphenyletherdisulfonat in Wasser (Emulgator)
- 50 g: 20 gew.-%ige Lösung des Umsetzungsprodukts von p-Isononylphenol mit ca. 50 Mol Ethylenoxid in Wasser (Emulgator)

### Zulauf 2:

- 100 g: entsalztes Wasser
- 3 g: Natriumpersulfat

Vernetzungsmittel V2 bis V4 wurden entsprechend hergestellt (Tabelle 3).

**Tabelle 3:**

| Zusammensetzung der Vernetzungsmittel in Gew.-% | | | | | |
|---|---|---|---|---|---|
| Vernetzungsmittel | EA | MMA | HEA | DAA | AAEM |
| V1 | 80 | 18 | | 2 | |
| V2 | 96 | | | 4 | |
| V3 | 77,7 | 17,4 | | | 4,9 |
| V4 | 96 | | 2 | 2 | |

- EA:: Ethylacrylat
- MMA:: Methylmethacrylat
- HEA:: Hydroxyethylacrylat
- DAA:: Diacetonacrylamid
- AAEM:: Acetoacetoxyethylmethacylat

Herstellung von Dispersionen, welche fremdvernetzende Copolymerisate enthalten (D1 - D4).

Die Herstellung erfolgt wie unter V1 beschrieben, wobei die Zuläufe folgende Zusammensetzung hatten:

### Zulauf 1: (dieser Zulauf wird während der Polymerisation gerührt)

- 107,5 g: entsalztes Wasser
- 400 g: Ethylacrylat
- 90 g: Methylmethacrylat
- 5 g: eines Oximethers gemäß Tabelle 4
- 50 g: 20 gew.-%ige Lösung des Natriumsalzes vom p-Dodecyldiphenyletherdisulfonat in Wasser (Emulgator)
- 50 g: 20 gew.-%ige Lösung des Umsetzungsprodukts von p-Isononylphenol mit ca. 50 Mol Ethylenoxid in Wasser (Emulgator)

### Zulauf 2:

- 100 g: entsalztes Wasser
- 3 g: Natriumpersulfat

**Tabelle 4:**

| Dispersionen fremdvernetzender Copolymerisate D1 - D4 | |
|---|---|
| Dispersion | Oximether (aus Tabelle 1) |
| D 1 | 1 |
| D 2 | 7 |
| D 3 | 8 |
| D 4 | 10 |

Herstellung von Dispersionen, welche selbstvernetzende Copolymerisate enthalten (D5 - D8).

Die Herstellung erfolgt wie unter V1 beschrieben, wobei die Zuläufe folgende Zusammensetzung hatten:

### Zulauf 1: (dieser Zulauf wird während der Polymerisation gerührt)

- 107,5 g: entsalztes Wasser
- 400 g: Ethylacrylat
- 90 g: Methylmethacrylat
- 50 g: 20 gew.-%ige wäßrige Diacetonarylamid-Lösung
- 5 g: eines Oximethers gemäß Tabelle 5
- 50 g: 50 g 20 gew.-%ige Lösung des Natriumsalzes vom p-Dodecyldiphenyletherdisulfonat in Wasser (Emulgator)
- 50 g: 20 gew.-%ige Lösung des Umsetzungsprodukts von p-Isononylphenol mit ca. 50 Mol Ethylenoxid in Wasser (Emulgator)

### Zulauf 2:

- 100 g: entsalztes Wasser
- 3 g: Natriumpersulfat

**Tabelle 5:**

| Dispersionen selbstvernetzender Copolymerisate D5 - D8 | |
|---|---|
| Dispersion | Oximether (aus Tabelle 1) |
| D 5 | 1 |
| D 6 | 7 |
| D 7 | 8 |
| D 8 | 10 |

Prüfung der Dispersionen auf Vernetzbarkeit und anwendungstechnische Eigenschaften

### Vernetzbarkeit (Prüfung durch Quellungsverhalten)

Es wurden die erhaltenen Dispersionen der Vernetzungsmittel V1 - V4 jeweils mit den fremdvernetzenden Dispersionen D1 - D4 im Gewichtsverhältnis 1:1 gemischt (Tabelle 4).

Je 200 g der erhaltenen Mischungen bzw. 200 g der selbstvernetzenden Dispersionen D5 - D8 wurden verfilmt und die Filme 1 Woche bei Raumtemperatur getrocknet. Anschließend wurde das Quellungsverhalten als Maß für den Vernetzungsgrad dieser Filme in Tetrahydrofuran untersucht, indem ca. 1 g der verfilmten Proben in Tetrahydrofuran 2 Tage gelagert und die Lösungsmittelaufnahme in % gemessen wurde (Ergebnisse in Tab. 6).

Bei vernetzten Polymeren kommt es durch Aufnahme von Lösungsmittel zur Quellung. Mit steigendem Vernetzungsgrad wird die Quellung geringer, da weniger Lösungsmittel vom eng vernetzten Polymer aufgenommen werden kann. Nicht oder kaum vernetzte Polymere werden durch Lösungsmittel zum großen Teil gelöst oder quellen bei Vorhandensein einer geringen Anzahl von Vernetzungsstellen übermaßig stark an.

### Herstellung von Verbundfolien und Prüfung der Schälfestigkeit

Die obigen Mischungen und Dispersionen D5 - D8 wurden mit einer Trockenschichtdicke von 3 g/m auf verschiedene auf 50°C erwärmte Folien (Polyethylentherephthalat: PETP; Polyamid: PA; Polyvinylchlorid: PVC;) gerakelt, und nach 20 sek. mit einer Polyethylenfolie PE (coronavorbehandelt) kaschiert. Anschließend wurden die Folien 7 Tage bei Raumtemperatur und Normalklima gelagert und danach in 2 cm breite Streifen zerschnitten. Diese Streifen wurden dann bei 23°C im Winkel von 180°C mit einer Geschwindigkeit von 100 m/min abgezogen. Es wurde die Schälkraft in N bei den 2 cm breiten Streifen bestimmt (Tabelle 6).

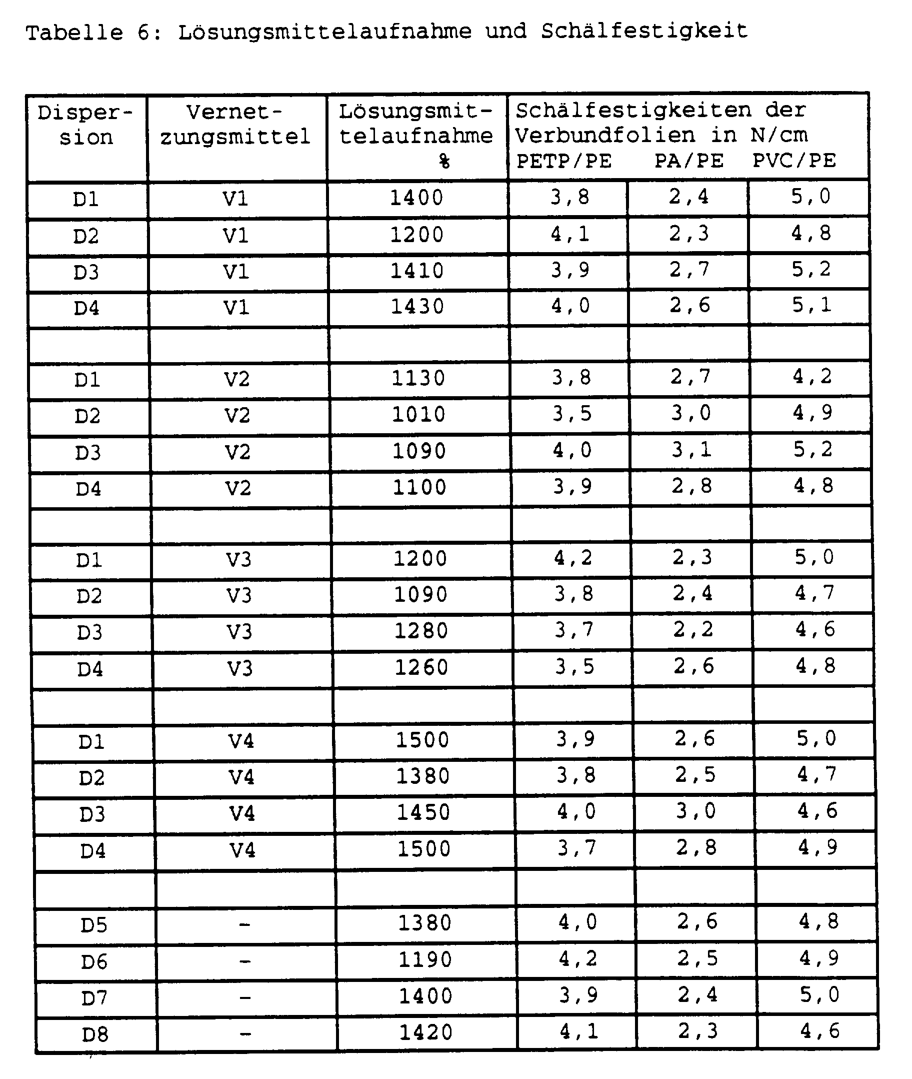

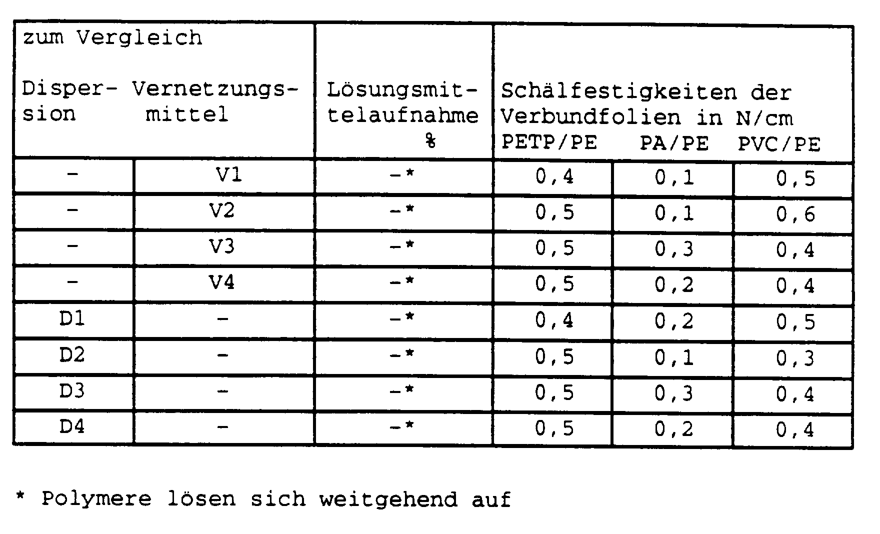

## Patentansprüche

1. Copolymerisierbare Oximether der allgemeinen Formel 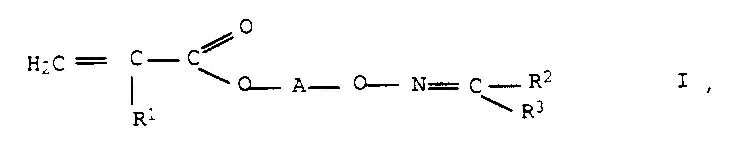 worin A für ein zweiwertiges Bindeglied steht, R¹ ein Wasserstoffatom oder eine C₁- bis C₄-Alkylgruppe sein kann und R und R³ unabhängig voneinander für einen C₁-bis C₁₀-Alkyl-, einen C₁-C₁₀-Alkoxy-, einen C₅- bis C₁₀-Cycloalkyl- oder einen C₅- bis C₁₀-Arylrest, welche auch 1 - 3 nicht benachbarte Stickstoff-, Sauerstoff- oder Schwefelatome als Heteroatome in der Kohlenstoffkette oder im Kohlenstoffring enthalten können und durch ein bis drei C₁- bis C₄-Alkyl- oder -Alkoxygruppen substituiert sein können, stehen, R oder R³ für ein Wasserstoffatom stehen können oder R und R³ gemeinsam eine Brücke aus 3 bis 14 Kohlenstoffatomen bilden, wobei ein Teil der Kohlenstoffatome auch Bestandteil eines aromatischen Rings sein kann.

2. Verfahren zur Herstellung von copolymerisierbaren Oximethern gemäß Anspruch 1, dadurch gekennzeichnet, daß Oximetheralkohole der allgemeinen Formel 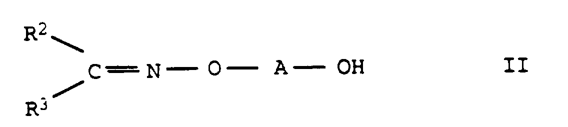 mit Acrylsäurechloriden der allgemeinen Formel 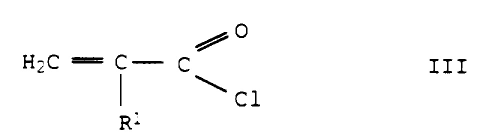 oder Acrylsäureanhydriden der allgemeinen Formel 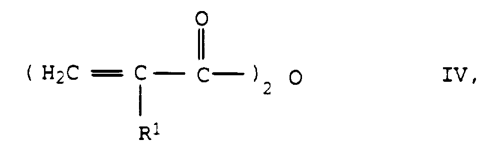 in Gegenwart einer Base umgesetzt werden.

3. Copolymerisate, enthaltend 0,01 bis 30 Gew.-% der Oximether gemäß Anspruch 1.

4. Copolymerisate nach Anspruch 3, enthaltend
a) 0,01 bis 30 Gew.-% eines Oximethers der Formel I
b) 30 bis 99,99 Gew.-% mindestens eines C₁ bis C₂₀-Alkyl(meth-)acrylats, eines Vinylesters von 1 bis 20 C-Atome enthaltenden Carbonsäuren, eines Vinylaromaten mit bis zu 20 C-Atomen, eines ethylenisch ungesättigten Nitrils mit 3-6 C-Atomen, eines Vinylhalogenids oder eines nichtaromatischen Kohlenwasserstoffs mit 4 bis 8 C-Atomen und mindestens 2 konjugierten Doppelbindungen,
c) 0 bis 30 Gew.-% mindestens eines Comonomeren mit mindestens einer Keto- oder Aldehydgruppe und
d) 0 bis 50 Gew.-% mindestens eines weiteren Monomeren,
wobei das Copolymerisat eine Glasübergangstemperatur zwischen -60 und +140°C hat.

5. Verwendung von Copolymerisaten gemäß Anspruch 3 oder 4 als Beschichtungsmittel.

6. Verwendung von Copolymerisaten gemäß Anspruch 3 oder 4 als Klebstoffe.

## Claims

1. A copolymerizable oxime ether of the formula 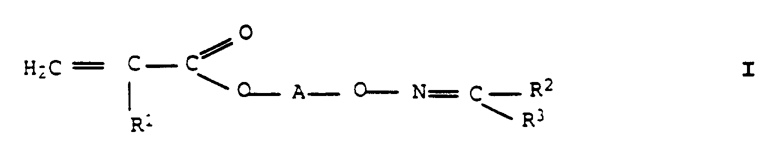 where A is a divalent link, R¹ may be hydrogen or C₁-C₄-alkyl and R and R³ independently of one another are each C₁-C₁₀-alkyl, C₁-C₁₀-alkoxy, C₅-C₁₀-cycloalkyl or C₅-C₁₀-aryl, each of which may furthermore contain 1-3 nonadjacent nitrogen, oxygen or sulfur atoms as hetero atoms in the carbon chain or in the carbon ring and may be substituted by from one to three C₁-C₄-alkyl or C₁-C₄-alkoxy groups, R or R³ may be hydrogen or R and R³ together form a bridge of 3 to 14 carbon atoms, where some of the carbon atoms may also be part of an aromatic ring.

2. A process for the preparation of a copolymerizable oxime ether as claimed in claim 1, wherein an oxime ether alcohol of the formula 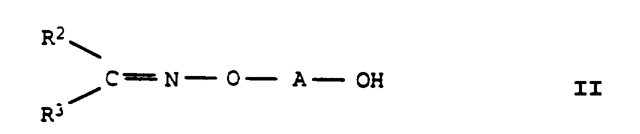 is reacted with an acryloyl chloride of the formula 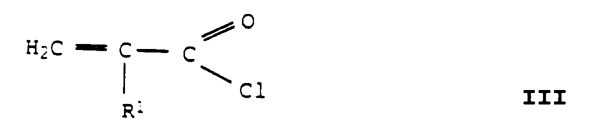 or an acrylic anhydride of the formula 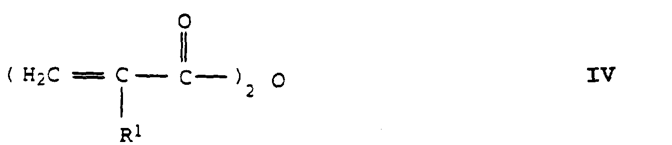 in the presence of a base.

3. A copolymer containing from 0.01 to 30% by weight of an oxime ether as claimed in claim 1.

4. A copolymer as claimed in claim 3, containing
a) from 0.01 to 30% by weight of an oxime ether of the formula I,
b) from 30 to 99.99% by weight of at least one C₁-C₂₀-alkyl (meth)acrylate, one vinyl ester of carboxylic acid of 1 to 20 carbon atoms, one vinyl aromatic of up to 20 carbon atoms, one ethylenically unsaturated nitrile of 3-6 carbon atoms, one vinyl halide or one nonaromatic hydrocarbon having 4 to 8 carbon atoms and at least 2 conjugated double bonds,
c) from 0 to 30% by weight of at least one comonomer having at least one keto or aldehyde group and
d) from 0 to 50% by weight of at least one further monomer,
the copolymer having a glass transition temperature of from -60 to +140°C.

5. Use of a copolymer as claimed in claim 3 or 4 as a coating material.

6. Use of a copolymer as claimed in claim 3 or 4 as an adhesive.

## Revendications

1. Ether-oximes copolymérisables de formule générale 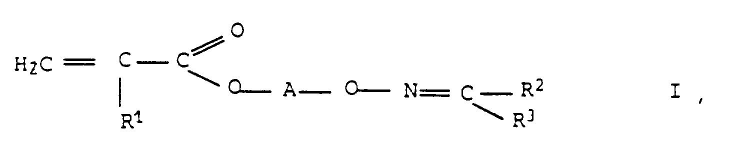 dans laquelle A est mis pour un maillon de liaison bivalent, R¹ peut être un atome d'hydrogène ou un groupement alkyle en C₁-C₄ et R et R³ sont mis chacun, indépendamment l'un de l'autre, pour un reste alkyle en C₁-C₁₀, alcoxy en C₁-C₁₀, cycloalkyle en C₅-C₁₀ ou aryle en C₅-C₁₀ qui peut aussi contenir 1 à 3 atomes d'azote, d'oxygène ou de soufre non voisins en tant qu'hétéroatomes dans la chaîne carbonée ou dans le cycle carboné et peut être substitué par 1 à 3 groupements alkyle ou alcoxy en C₁-C₄, R ou R³ peut être mis pour un atome d'hydrogène ou R et R³ forment ensemble un pont de 3 à 14 atomes de carbone, une partie des atomes de carbone pouvant aussi entrer dans la constitution d'un cycle aromatique.

2. Procédé de préparation d' éther-oximes copolymérisables selon la revendication 1, caractérisé en ce que des éther-oxime-alcools de formule générale 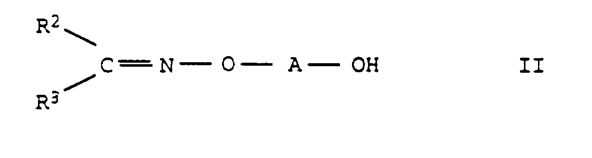 sont mis en réaction avec des chlorures d'acide acrylique de formule générale 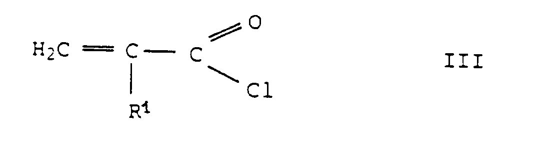 ou des anhydrides d'acide acrylique de formule générale 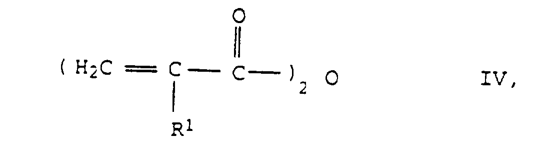 en présence d'une base.

3. Copolymères, contenant 0,01 à 30% en poids des éther-oximes selon la revendication 1.

4. Copolymères selon la revendication 3, contenant
a) 0,01 à 30% en poids d'un éther-oxime de formule I,
b) 30 à 99,99% en poids d'au moins un (méth)acrylate d'alkyle en C₁-C₂₀, un ester vinylique d'acide carboxylique contenant 1 à 20 atomes de carbone, un composé vinylaromatique contenant jusqu'à 20 atomes de carbone, un nitrile à insaturation éthylénique à 3-6 atomes de carbone, un halogénure de vinyle ou un hydrocarbure non aromatique contenant 4 à 8 atomes de carbone et au moins 2 doubles liaisons conjuguées,
c) 0 à 30% en poids d'au moins un comonomère contenant au moins un groupement céto ou aldéhyde et
d) 0 à 50% en poids d'au moins un autre monomère,
le copolymère ayant une température de transition vitreuse comprise entre -60 et +140°C.

5. Utilisation de copolymères selon la revendication 3 ou 4 comme produits d'enduction.

6. Utilisation de copolymères selon la revendication 3 ou 4 comme colles.
